# EUROPEAN PATENT SPECIFICATION

(11) **EP 2 172 196 B1**
(45) Date of publication and mention of the grant of the patent: **25.03.2020**
(21) Application number: 08765784.7
(22) Date of filing: 20.06.2008
(51) Int. Cl.: A61K 31/122, A61K 8/34, A61K 8/35, A61K 8/37, A61K 8/55, A61K 8/60, A61K 8/64, A61K 47/10, A61K 47/14, A61K 47/24, A61K 47/26, A61K 47/42, A61K 9/16, A61K 9/20, A61Q 19/00, A23K 20/111, A23K 20/147, A23K 20/10, A23L 33/10, A23K 20/163

(54) **COMPOSITION CONTAINING COENZYME Q10**
COENZYM Q10 ENTHALTENDE ZUSAMMENSETZUNG
COMPOSITION CONTENANT UNE COENZYME Q10

(30) Priority: 22.06.2007 JP 2007165176; 05.11.2007 US 996157 P
(43) Date of publication of application: 07.04.2010
(73) Proprietor: Kaneka Corporation, Osaka (JP)
(72) Inventor: IKEHARA, Toshinori, Takasago-shi Hyogo 676-8688 (JP); SAKOGAWA, Takayuki, Takasago-shi Hyogo 676-8688 (JP)
(74) Representative: J A Kemp LLP
(86) International application number: PCT/JP2008/061364
(87) International publication number: WO 2009/001787

(56) References cited:
- EP-A1- 1 591 020
- EP-A1- 1 782 803
- EP-A1- 1 829 538
- EP-A2- 0 065 193
- WO-A1-2005/035477
- JP-A- 57 054 116
- JP-A- 2003 169 630
- JP-A- 2003 321 352
- JP-A- 2004 075 664
- JP-A- 2005 524 635
- US-A1- 2005 092 969
- DATABASE CAPLUS [Online] XP008118662 Retrieved from STN Database accession no. (2007:597834) & CN 1 969 833

## Description

### [Technical Field]

The present invention relates to a composition comprising coenzyme Q10. More specifically, the present invention relates to an emulsified powder comprising coenzyme Q10, having powder characteristics allowing easy handling and high oral absorbability.

### [Background Art]

Coenzyme Q10 is a physiological component present as a constituent component of the mitochondrial electron transport system in the cell of the living body. It functions as a transport component in the electron transport system by repeating oxidation and reduction in the living body. Coenzyme Q10 is known to show energy production, membrane stabilization and antioxidant activity in the living body, and has a high degree of usability. Coenzyme Q10 occurs in two forms, the oxidized form and the reduced form, and it is known that, in the living body, usually about 40 to 90% of the coenzyme exists in the reduced form. Of coenzymes Q10, oxidized coenzyme Q10 (aka. ubiquinone or ubidecarenone) is widely used in the pharmaceutical field as a drug for congestive heart failure. Besides the pharmaceutical use, it is widely used as an agent for oral preparation and a skin preparation, as a nutritional product or a nutritional supplement, like vitamin. More recently, oxidized coenzyme Q10 has been approved for use as a food, and it is drawing attention as a material for health foods. It is known, however, that coenzyme Q10 poses problems of poor powder fluidity and tableting trouble when used as is for hard capsules and tablets. In addition, coenzyme Q10 is an oil-soluble crystalline powder having a melting point of about 48°C, and is known to show extremely low absorbability by oral ingestion since it is sparingly soluble in water.

To solve these problems, a wide variety of technical proposals have been made to date. For example, it has been proposed to prepare an emulsified product containing coenzyme Q10 by using a synthetic emulsifier such as glycerol fatty acid ester, sucrose fatty acid ester and powderize the product by a spray dry method (patent references 1 - 3). Such production method achieves superior emulsion stability. However, it requires addition of a large amount of excipient for production of a dry powder and, as a result, the content of coenzyme Q10 cannot be high.

In addition, a method of using a water-soluble polymer without using the above-mentioned synthetic emulsifier is also suggested. For example, a method of dispersing/emulsifying coenzyme Q10 in an aqueous solution containing a water-soluble polymer in the presence of an organic acid is disclosed (patent reference 4). Furthermore, a nutrient composition comprising coenzyme Q10, casein sodium and dextrin is disclosed (patent reference 5). Even these methods are associated with problems in the workability since the powder property decreases when the content of coenzyme Q10 is increased. Moreover, a solid composition containing coenzyme Q10 at a high content is disclosed (patent reference 6). However, the starch octenylsuccinate to be used is a processed starch obtained by reacting starch and octenylsuccinic anhydride, and the use thereof is not desired in many cases.

There has been a demand for a powder preparation having a high coenzyme Q10 content, which is excellent in the water-dispersibility, water-solubility and workability, and is substantially free of a synthetic emulsifier.
[patent reference 1] JP-A-59-51214
[patent reference 2] JP-A-2000-212066
[patent reference 3] JP-A-2003-238396
[patent reference 4] JP-A-2003-55203
[patent reference 5] JP-B-3575762
[patent reference 6] WO2006/022187

Compositions containing coenzyme Q10 are also disclosed in EP-A-1,591,020, EP-A-1,782,803 and EP-A-1,829,538.

A method of stabilising a compound having a quinone skeleton and stabilised composition are disclosed in US 2005/0092969 and WO 2005/035477.

JP-A-57/054116 discloses a coenzyme Q10 composition produced by a fluidised bed granulation method.

### [Disclosure of the Invention]

### [Problems to be Solved by the Invention]

To solve the above-mentioned problems, the present invention proposes a composition and a powder, each comprising coenzyme Q10, which are applicable to the fields of foods, food with nutrient function claims, food for specified health uses, nutritional supplement, nutritional product, animal drug, drinks, feed, cosmetic, quasi-drug, pharmaceutical product, therapeutic drug, prophylactic drug and the like and superior not only in the water-dispersibility/solubility but also in the recovery rate during production, workability such as powder flowability and the like, as well as tabletability.

### [Means of Solving the Problems]

The present inventors have conducted intensive studies in an attempt to solve the above-mentioned problems and found that a powder comprising coenzyme Q10 dispersed in a matrix comprising casein sodium and a saccharide other than polysaccharides at a particular ratio, is a composition superior in the water-dispersibility/solubility, workability and tabletability, which resulted in the completion of the present invention.

Accordingly, the present invention provides the following.
[1] A coenzyme Q10-containing composition comprising 1-70 wt % of coenzyme Q10 (A), 15-94 wt % of casein (B), and at least 5 wt % of saccharide (C) other than polysaccharides, wherein the saccharide (C) other than polysaccharides is at least one kind selected from the group consisting of erythritol, trehalose, and an oligosaccharide having 3 to 5 sugars.
[2] The composition of [1] above, further comprising 0.1-20 wt % of surfactant (D).
[3] The composition of [1] or [2] above, wherein the weight ratio of coenzyme Q10 (A) and casein (B) is within the range of 1:10 - 5:1 and the weight ratio of casein (B) and saccharide (C) other than polysaccharides is within the range of 1:10 - 10:1.
[4] The composition of any one of [1] to [3] above, wherein the casein (B) is casein sodium.
[5] The composition of any one of [2] to [4] above, wherein the surfactant (D) is at least one kind selected from the group consisting of glycerol fatty acid esters, sucrose fatty acid esters, sorbitan fatty acid esters, lecithins and saponins.
[6] The composition of any one of [1] to [5] above, which is a powder.
[7] Non-therapeutic use of the composition of any one of [1] to [6] above in a food, a cosmetic or a feed .
[8] A process of manufacturing a food, a pharmaceutical product, a cosmetic or a feed, comprising adding a composition as defined in any one of [1] to [6] above.
[9] A production method of a coenzyme Q10-containing powder comprising 1 - 70 wt % of coenzyme Q10 (A), 15 - 94 wt % of casein (B), and at least 5 wt % of saccharide (C) other than polysaccharides, wherein the saccharide (C) other than polysaccharides is at least one kind selected from the group consisting of erythritol, trehalose, and an oligosaccharide having 3 to 5 sugars, which method comprises preparing an oil-in-water emulsified composition comprising coenzyme Q10 (A) as an oil phase and an aqueous solution containing casein (B) and saccharide (C) other than polysaccharides as an aqueous phase, and removing water.

### [Effect of the Invention]

According to the present invention, a coenzyme Q10-containing composition or powder superior in water-dispersibility/solubility, workability and tabletability can be provided, which is advantageous for the production of food, food with nutrient function claims, food for specified health uses, nutritional supplement, nutritional product, animal drug, drinks, feed, cosmetic, quasi-drug, pharmaceutical product, therapeutic drug, prophylactic drug.

### [Best Mode for Carrying out the Invention]

The embodiment of the present invention is explained in detail in the following.

The coenzyme Q10-containing composition of the present invention is a composition comprising 1-70 wt % of coenzyme Q10 (A), 15 - 94 wt % of casein (B), and at least 5 wt % of saccharide (C) other than polysaccharides (hereinafter sometimes to be referred to as the composition of the present invention). One embodiment of the composition of the present invention is a powder containing the above-mentioned (A), (B) and (C) (sometimes to be referred to as the powder of the present invention). Specifically, it is an emulsified powder obtained by dispersing coenzyme Q10 in a matrix comprising casein and saccharide. The emulsified powder here means a powder that becomes an oil-in-water emulsion when dissolved in water.

The coenzyme Q10 (A) to be used in the present invention is not particularly limited, and any of oxidized coenzyme Q10, reduced coenzyme Q10, and a mixture thereof can be used. Oxidized coenzyme Q10 is represented by the following formula (1), and reduced coenzyme Q10 is represented by the following formula (2): 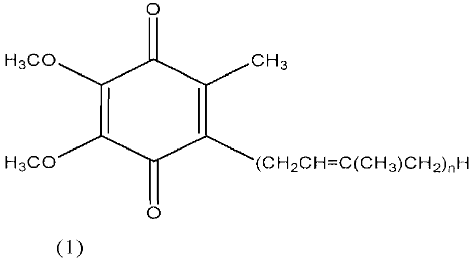 wherein n=10. 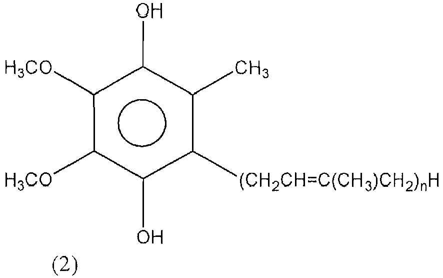 wherein n=10.

The content of coenzyme Q10 (A) in the composition of the present invention is generally within the range of 1 - 70 wt %, preferably 5 - 70 wt %, more preferably 10 - 50 wt %. In the composition of the present invention, when the content of coenzyme Q10 in the composition is less than 1 wt %, a large amount of the coenzyme Q10-containing composition needs to be ingested for oral administration of a given amount of coenzyme Q10.

The casein (B) to be used in the composition of the present invention is not particularly limited as long as it is acceptable for foods, cosmetics, pharmaceutical products and the like. Any of caseins such as α-casein, β-casein, κ-casein and the like, salts of casein such as casein sodium, casein calcium and other casein derivatives can be used, and mixtures thereof can be used. Particularly, casein sodium, which is widely used for foods and superior in emulsifying capacity, is preferable. The content of casein (B) in the composition of the present invention is 15 - 94 wt %, preferably not less than 20 wt %. When the content of casein in the composition of the present invention is less than 10 wt %, the emulsifiability is not sufficient. Furthermore, when the content exceeds 94 wt %, the contents of coenzyme Q10 and saccharide other than polysaccharides decrease relatively, and necessary amounts of these components cannot be contained.

Saccharide (C) other than polysaccharide to be used for the powder of the present invention is comparatively low molecular weight saccharide. Erythritol may be used as a monosaccharide-derived sugar alcohol, trehalose may be used as a disaccharide, or an oligosaccharide having 3 to 5 sugars may be used.

The content of the saccharide (C) other than polysaccharides in the composition of the present invention is at least 5 wt %, preferably 5-40 wt %, more preferably 10 - 30 wt %. When the content of saccharide (C) other than polysaccharides in the composition of the present invention is less than 5 wt %, the flowability of the powder is insufficient.

While the proportion of each component in the coenzyme Q10-containing composition of the present invention is not particularly limited, the following range is preferable. The weight ratio of coenzyme Q10 (A) and casein (B) ((A):(B)) is preferably within the range of 1:10 - 5:1, more preferably 1:5 - 3:1. When the proportion of casein (B) relative to coenzyme Q10 is less than 1/10, coenzyme Q10 may not be emulsified sufficiently, and when the amount of casein (B) is high, the content of saccharide (C) other than polysaccharides decreases to often degrade the powder flowability.

While the weight ratio of casein (B) and saccharide (C) other than polysaccharides ((B):(C)) is determined in consideration of emulsifiability and powder flowability, it is preferably within the range of 1:10 - 10:1, more preferably 1:5 - 5:1, most preferably 1:2 - 2:1. When the proportion of casein (B) relative to saccharide (C) other than polysaccharides is too high, the emulsifiability is good but the powder flowability tends to decrease. On the other hand, when the proportion of saccharide (C) is too high, the powder flowability is good but the emulsifiability decreases.

In the composition of the present invention, while casein is used as an emulsifier, other surfactant (D) acceptable for foods, cosmetics, pharmaceutical products and the like may be used in combination with casein. As such surfactant (D), for example, glycerol fatty acid esters, sucrose fatty acid esters, sorbitan fatty acid esters, lecithins and saponins can be mentioned. When the content of surfactant (D) in the composition of the present invention is not less than 0.1 wt%, a composition more superior in the emulsification stability can be obtained. From this viewpoint, the content of surfactant (D) in the composition of the present invention is generally 0.1 - 20 wt%, preferably 0.1 - 15 wt%, more preferably 1 - 10 wt%. When the content of surfactant (D) exceeds 20 wt%, the emulsifiability is good but the powder flowability tends to decrease.

As the aforementioned glycerol fatty acid esters, for example, fatty acid and organic acid esters of monoglycerol, polyglycerol fatty acid esters, polyglycerin condensed ricinoleate can be mentioned.

As the fatty acid and organic acid esters of monoglycerol, for example, stearic acid and citric acid ester of monoglycerol, stearic acid and acetic acid ester of monoglycerol, stearic acid and succinic acid ester of monoglycerol, caprylic acid and succinic acid ester of monoglycerol, stearic acid and lactic acid ester of monoglycerol, stearic acid and diacetyltartaric acid ester of monoglycerol can be mentioned.

As the polyglycerol fatty acid ester, for example, one having an average degree of polymerization of polyglycerin of 2 - 10, wherein the constituent fatty acid has 6 to 22 carbon atoms, can be mentioned. When emulsification stability is to be imparted, polyglycerin fatty acid monoester is preferable. More preferable example is decaglycerol fatty acid monoester, specifically decaglycerol monolaurate, decaglycerol monomyristate, decaglycerol monooleate, decaglycerol monopalmitate, and decaglycerol monostearate.

Examples of the polyglycerin condensed ricinoleate include one having an average degree of polymerization of polyglycerin of 2 - 10, wherein the average degree of condensation of polyricinoleic acid (average number of condensation of ricinoleic acid) is 2 to 4.

As the aforementioned sucrose fatty acid esters, one wherein one or more hydroxyl groups of sucrose is/are each esterified with fatty acid having 6 to 22, preferably 12 to 18, carbon atoms can be mentioned. Further preferred is fatty acid monoester, specifically sucrose monostearate.

Examples of the aforementioned sorbitan fatty acid esters include sorbitans wherein one or more hydroxyl groups thereof are esterified by fatty acids having 6 to 22, preferably 12 to 18, carbon atoms, and polyoxyethylene sorbitan fatty acid esters wherein polyoxyethylene is added to hydroxyl groups of sorbitans, further preferably polyoxysorbitan monooleate.

Examples of the aforementioned lecithins include egg-yolk lecithin, soybean lecithin, enzymatically decomposed lecithin, phosphatidylcholine, phosphatidylethanolamine, phosphatidylserine, sphingomyelin, dicetyl phosphate, stearylamine, phosphatidylglycerol, phosphatidic acid, phosphatidylinositolamine, cardiolipin, ceramide phosphorylethanolamine, ceramide phosphoryl glycerol and a mixture thereof and the like. Of these, enzymatically decomposed lecithin is preferable. The enzymatically decomposed lecithin is, for example, preferably obtained by reacting phospholipase A2 with egg-yolk lecithin or soybean lecithin, and one containing lysolecithin as a main ingredient is preferable.

As the aforementioned saponins, for example, enju saponin, quillaja saponin, soybean saponin, yucca saponin can be mentioned.

Furthermore, it is possible to add other oil-soluble components to the coenzyme Q10-containing composition of the present invention, as long as the powder property thereof and dispersibility in water are not influenced. As such oil-soluble component, for example, edible fat and oil, fatty acid and ester derivatives thereof, wax, oil-soluble vitamins, carotenoids, plant extracts can be mentioned.

The aforementioned edible fat and oil is not particularly limited and, for example, it may be natural fat and oil from plant or animal, or synthetic fat and oil, processed fat and oil. More preferably, it is acceptable for foods, cosmetics, pharmaceutical agents. Examples of the vegetable fat and oil include coconut oil, palm oil, palm kernel oil, flaxseed oil, camellia oil, brown rice germ oil, rape seed oil, rice oil, peanuts oil, corn oil, wheat germ oil, soy bean oil, perilla oil, cottonseed oil, sunflower kerel oil, kapok oil, evening primrose oil, shea butter, sal butter, cacao butter, sesame oil, safflower oil, olive oil, pomegranate oil, bitter gourd oil and the like, and examples of animal fats and oils include lard, milk fat, fish oil, beef fat. In addition, medium chain triglyceride wherein each fatty acid has a carbon number of 6 - 12, preferably 8 - 12, fats and oils obtained by processing them by fractionation, hydrogenation, transesterification etc. and partial glycerides thereof like can also be mentioned. Needless to say, a mixture of them may be used.

Examples of the aforementioned fatty acid and ester derivatives thereof include, but are not limited to, saturated fatty acids such as caprylic acid, capric acid, lauric acid, myristic acid, palmitic acid, stearic acid, and behenic acid; unsaturated fatty acids such as oleic acid, linoleic acid, conjugated linoleic acid, linolenic acid, punicic acid, docosahexaenoic acid, docosapentaenoic acid, and eicosapentaenoic acid; special fatty acids having an SS bond in the molecular structure thereof, such as α lipoic acid, and esters thereof, for example, methyl esters and ethyl esters thereof.

Examples of the aforementioned wax include wax for food such as bees wax, rhus succedanea fruit wax, candelilla wax, rice bran wax, carnauba wax, snow wax.

Examples of the aforementioned oil-soluble vitamins include, but are not limited to, vitamin A, vitamin D, vitamin E, vitamin K, tocotrienol and derivatives thereof.

The aforementioned carotenoids include, but are not limited to, at least one kind selected from the group consisting of carotenes, xanthophylls, and derivatives thereof; specifically, carotenes are exemplified by α carotene, β carotene, γ carotene, δ carotene, ε carotene, and licopene, and xanthophylls are preferably exemplified by lutein, zeaxanthin, canthaxanthin, fucoxanthin, anthraxanthin, violaxanthin, and astaxanthin.

The aforementioned plant extract includes, but is not limited to, one obtained by extracting a plant generally used for foods with an organic solvent. Examples thereof include a hydrophobic extract obtained by extracting a plant such as licorice, turmeric, perilla, clove, cinnamon, ginger, lemongrass, peppermint, *dokudami* (Houttuyania cordata), coix seed, rice bran, cornflower, fennel, boxthorn, zanthoxylum, nasturtium, dioscorea, sanryo, kinkaran (Tinospora capillipes), *amachazuru* (Gynostema pentaphyllum), thuja, hakutouou (Pulsatilla chinensis), parsley, onion, nutmeg, wild-rice, gluten feed, *konnyaku tobiko* (konnyaku byproduct powder), paprika, horseradish, lemon, capsicum, sesame, spearmint, leaf mustard and the like or a plant processed product with an organic solvent such as ethanol, acetone, hexane and the like. The active ingredient of a hydrophobic extract is, for example, polyphenols, terpenes. Of these, a preferable plant extract is an ethanol extract of licorice (main component; licorice polyphenol). These plant extracts may be used in the form of a solution in fat and oil (for example, medium chain triglyceride).

In the coenzyme Q10-containing composition of the present invention, a water-soluble polymer other than caseins can also be used in combination with caseins according to various objects. Such water-soluble polymer is not particularly limited as long as it is acceptable for use as foods, cosmetics, pharmaceutical products with preference given to those particularly acceptable for food. For example, water-soluble polymer such as gum arabic, gum ghatti, guar gum, gelatine, agar, starch, modified starch, pectin, carrageenan, dried albumen, curdlan, alginic acids, soybean polysaccharides, pullulan, celluloses, xanthan gum, carmellose salt (carmellose sodium, carmellose calcium etc.), sugar ester of higher fatty acid, tragacanth, protein other than casein, polyvinylpyrrolidone can be used.

In addition, the coenzyme Q10-containing composition of the present invention may comprise various additives and water-soluble active ingredients useful for a wide variety of purposes in foods, cosmetics, and pharmaceuticals, added according to the respective purposes, as long as the properties thereof are not affected.

Examples of such additives include excipients such as crystalline cellulose, calcium phosphate, calcium sulfate and disintegrants such as calcium citrate, calcium carbonate, sodium hydrogencarbonate, dextrin, crystalline cellulose, carboxymethylcellulose, tragacanth, alginic acid lubricants such as talc, magnesium stearate, polyethylene glycol, silica, hydrogenated oil and antiblocking agents such as stearic acid, light anhydrous silicic acid, hydrated silicon dioxide and absorption promoters such as higher alcohols, higher fatty acids and the like, solubilizing agents such as fumaric acid, succinic acid, malic acid and stabilizers such as benzoic acid, sodium benzoate, ethyl p-oxybenzoate, bees wax.

Examples of the water-soluble active ingredients include, but are not limited to, water-soluble vitamins, amino acids, organic acids, peptides, proteins, nucleic acids, water-soluble polyphenols.

Examples of the aforementioned water-soluble vitamins include vitamin C, vitamin B, folic acid, nicotinic acid, nicotinic acid amide, pantothenic acid, pyrroloquinolinequinone, and isomers and derivatives thereof.

Examples of the aforementioned amino acids include alanine, β-alanine, arginine, asparagine, aspartic acid, cysteine, N-acetylcysteine, selenocysteine, cystine, glutamine, glutamic acid, glycine, histidine, isoleucine, leucine, lysine, pyrrololysine, hydroxylysine, methionine, phenylalanine, proline, hydroxyproline, serine, O-phosphoserine, threonine, tryptophan, tyrosine, valine, thyroxine, desmosine, ornithine, creatine, γ-aminobutyric acid, theanine, taurine, and isomers and derivatives thereof. L-carnitine, a special amino acid, and pharmacologically acceptable salts thereof, such as tartrates and fumarates, acetyl-L-carnitine, and propionyl-L-carnitine are also suitable. It is also suitable to use peptides of two or more of these amino acids bound together; examples include, but are not limited to, cysteine peptides such as glutathione, soybean peptide, sesame peptide, silk peptide, sardine peptide, collagen peptide and casein phosphopeptide. Examples of organic acids other than amino acids include, but are not limited to, citric acid, malic acid, succinic acid, catechinic acid, picric acid, fumaric acid, maleic acid, and tartaric acid.

Examples of aforementioned water-soluble polyphenols include, but are not limited to, grape polyphenol, pine tree bark polyphenol, apple polyphenol, cacao polyphenol, and green tea polyphenol. These polyphenols occur as multi-ingredient systems; specifically, flavonoids are exemplified by isoflavons such as genistin, daidzein, and puerarin; flavonols such as quercetin, kaempferol, myricetin, and rutin; flavanones such as hesperidin, naringenin, proanthocyanidin, anthocyanin, cyanidin, delphinidin, malvidin, peonidin, petunidine and the like; flavanol such as epicatechin, epigallocatechin, epicatechin gallate, epigallocatechin gallate, and theaflavine; and flavons such as chrysin, apigenin, and luteolin. Lignans such as sesamin, sesamolin, sesaminol, and sesamol, or chlorogenic acid, gallic acid, ellagic acid, galangin, fisetin are also suitable.

Furthermore, minerals can be added as other useful ingredients; examples of minerals include, but are not limited to sodium, calcium, magnesium, zinc, iron, copper, selenium, chromium, manganese, iodine, molybdenum, and salts thereof.

Moreover, a light-fast ingredient may be further added. A light-fast ingredient that may be added is not particularly limited as long as it is a component used for foods, quasi-drugs, pharmaceutical products and the like and, for example, colorants used for soft capsules and hard capsules is preferable, with specific preference given to pigment such as titanium oxide, food colors, red iron oxide pigment, safflower pigment, annatto pigment, caramel pigment, gardenia pigment, tar pigment, chlorophyll and the like. The amount of the light-fast ingredient to be added is not particularly limited, as long as it does not influence the disintegration property and dispersibility/solubility of the obtained composition and is, for example, 0.01 - 10.0 wt%, preferably 0.1 - 5.0 wt%. When the amount of the addition is less than 0.01 wt%, the effect of stability to light may not be achieved, and when it exceeds 10.0 wt%, the disintegration property and dispersibility/solubility of the obtained composition may be affected.

Now, the production method of the composition of the present invention, specifically the powder of the present invention, is explained. The powder of the present invention is preferably obtained by, though not limited to, the following production method.

The production method of the coenzyme Q10-containing powder of the present invention comprises preparing an oil-in-water emulsified composition comprising coenzyme Q10 as an oil phase and an aqueous solution containing casein and saccharide other than polysaccharides as an aqueous phase, and removing water (hereinafter sometimes to be referred to as the production method of the present invention). Specifically, a preferable production method of a coenzyme Q10-containing powder comprises preparing an oil-in-water emulsified composition from coenzyme Q10 (A) (oil phase) and an aqueous solution (aqueous phase) containing casein (B) and saccharide (C) other than polysaccharides, and drying said oil-in-water emulsified composition to give an emulsified powder.

In the production method of the present invention, an oil phase can be prepared by a method including heating same to a temperature at which coenzyme Q10 melts (e.g., 50°C or above) and directly used. Alternatively, a method wherein other oily component is added as necessary and the mixture is mixed by stirring is most convenient and preferable. However, the method is not limited thereto. In addition, an aqueous phase is prepared by a method including dissolving casein (B), saccharide (C) other than polysaccharides and other aqueous component as necessary in water to give an aqueous solution. However, the method is not limited thereto.

In the production method of the present invention, the above-mentioned oil phase comprising coenzyme Q10 (A) and the above-mentioned aqueous phase which is an aqueous solution containing casein (B) and saccharide (C) other than polysaccharides are mixed to give an oil-in-water emulsified composition. The above-mentioned oil-in-water emulsified composition can be most conveniently and preferably prepared by, for example, heating an aqueous phase in advance to not less than 50°C, adding coenzyme Q10 heated similarly, and dispersing/emulsifying the coenzyme Q10 (A) ultrafinely to a desired average particle size using a known emulsification device such as a stirring homomixer, a high-pressure homogenizer etc. Alternatively, coenzyme Q10 or, where necessary, other oil component may be added to the aqueous phase heated in advance to not less than 50°C, coenzyme Q10 or other oil component is melted or dissolved in the aqueous phase and then emulsified. However, the method is not limited thereto.

The particle size of the emulsified coenzyme Q10 (A) in the above-mentioned oil-in-water emulsified composition is generally within the range of 10 - 5000 nm, preferably 10 - 1000 nm, more preferably 10 - 500 nm. An average particle size of the coenzyme Q10 (A) in the oil-in-water emulsified composition of greater than 5000 nm is unpreferable, since the recovery rate of the emulsified powder decreases in the drying step. On the other hand, an average particle size of the coenzyme Q10 (A) in the oil-in-water emulsified composition of smaller than 10 nm is unpreferable, since an ultra-high pressure in the emulsification step or an emulsifying operation under ultra-high speed stirring for a long time is necessary. The particle size of the above-mentioned emulsified oil component (A) in the oil-in-water emulsified composition can be measured by a commercially available laser diffraction/scattering type particle size distribution measurement device, a dynamic light scattering particle size distribution measurement device.

The emulsification method of the above-mentioned oil-in-water emulsified composition is not particularly limited as long as a desired particle size of the emulsified particles is achieved, and a mechanical emulsification method using a general emulsifier can be mentioned. As the apparatus to be used for a mechanical emulsification method, high-speed stirring emulsion machines such as TK homomixer (manufactured by Primix Corporation), Filmics (manufactured by Primix Corporation), Polytron (manufactured by KINEMATICA), Hiscotron (manufactured by microtec nition), Cleamix W-Motion (manufactured by M Technique Corporation) high-pressure emulsion machines such as microfluidizer (manufactured by Mizuho Industrial Co., Ltd.), Ultimizer system (manufactured by Sugino Machine Limited), nanomizer (manufactured by Yoshida Kikai Co., Ltd.), Manton-Gaulin homogenizer and the like, colloid mill, ultrasonication homogenizer can be mentioned. Besides the mechanical emulsification methods, membrane emulsion method, microchannel emulsion method, natural emulsion method, phase inversion emulsion method, gel emulsion method, D phase emulsion method can also be utilized. Of these, use of a high-pressure emulsion machine is preferable since it reduces the particle size of the emulsified particles, wherein the homogenized pressure is not less than 10 MPa, preferably not less than 20 MPa, more preferably not less than 50 MPa. The treatment may be performed plural times to afford a desired particle size of the emulsified particles.

In the production method of the present invention, a step for preparing an oil-in-water emulsified composition from oil phase and an aqueous phase is preferably performed at a temperature higher than the melting point of coenzyme Q10, which is generally within the range of 50 - 100°C, preferably 50 - 90°C, more preferably 60 - 80°C.

It is preferable to handle the above-mentioned oil-in-water emulsified composition at a concentration at which the viscosity of aqueous phase does not exceed 1 Poise, since the transferring property can be ensured.

In the production method of the present invention, a step for removing water from the above-mentioned oil-in-water emulsified composition is not particularly limited as long as it can remove water. As a general method, a method including drying can be mentioned. For example, spray-drying method, freeze-drying method, vacuum drying method, belt drying method, shelf drying method, drum drying method, liquid-drying method, spray cooler method can be mentioned. Of these, a spray-drying method most widely prevalent is particularly preferable from the aspects of producibility. In the case of a spray-drying method, the spray system is not particularly limited and, for example, 2 fluid nozzle, 3 fluid nozzle and atomizer can be mentioned. In this way, water is removed to give the powder of the present invention. The powder after recovery may be subjected to a classification operation to achieve a particle size desirable as a predetermined product.

The median size of an emulsified particle in the oil-in-water emulsion prepared by dissolving the thus-obtained powder of the present invention in water is generally within the range of 10 - 1000 nm, preferably 50 - 800 nm, more preferably 100 - 500 nm. In the present invention, the above-mentioned median size can be measured by a method including dissolving the obtained emulsified powder in water, and measuring the particle size of the obtained aqueous emulsion by a commercially available laser diffraction/scattering type particle size distribution measurement device, a dynamic light scattering particle size distribution measurement device.

In the production method of the present invention, when reduced coenzyme Q10 is used as coenzyme Q10, the step thereof is preferably operated under nitrogen atmosphere or under reduced pressure to prevent oxidation.

A powder containing coenzyme Q10, which is obtained by the present invention can also be processed into powder preparation, powder, granule, tablet, pill and filled in a hard capsule of gelatin, cellulose a soft capsule to give a supplement or a pharmaceutical product. Inasmuch as the powder shows good solubility in water, it can be dissolved in water to give an aqueous solution, which may be used as a drinkable preparation or cosmetic, or directly mixed with general foods, feed and the like. The powder of the present invention can be used for foods such as general foods, food with nutrient function claims, food for specified health uses, nutritional supplement, nutritional product, drinks; pharmaceutical products such as therapeutic drug, prophylactic drug, animal drug; quasi-drug, cosmetic, feed as mentioned above.

### [Examples]

The present invention is explained in more detail in the following by referring to Examples, which are not to be construed as limitative.

### (Example 1)

Casein sodium (manufactured by NIPPON SHINYAKU CO., LTD.; Hapuro, 5.8 g) and trehalose (manufactured by HAYASHIBARA; TREHA®, 5.8 g) were dissolved in distilled water (180 g) at 60°C to give an aqueous solution. Separately, oxidized coenzyme Q10 (manufactured by Kaneka Corporation; Kaneka Coenzyme Q10, 8.4 g) was melted at 60°C and added to the above-mentioned aqueous solution. The mixture was emulsified by POLYTRON (manufactured by KINEMATICA) at 10000 rpm × 10 min to give an oil-in-water emulsified composition. Then, the composition was sprayed and dried using a spray dryer (B-290; Nihon BUCHI K.K.) to give an oxidized coenzyme Q10-containing emulsified powder. As the property of the obtained emulsified powder, the flowability was good and attachment of the powder to the inside of the apparatus was extremely small. The obtained emulsified powder was dissolved in water, and the particle size of the obtained aqueous emulsion was measured using PARTICLE SIZE ANALYZER (dynamic light scattering particle size distribution measurement apparatus, LB-550, manufactured by HORIBA). As a result, the median size of the emulsified particles was 395 nm.

### (Example 2)

Using the same method as in Example 1 except that maltose (manufactured by Sanwa Cornstarch Co., Ltd.; Sunmalto S, 5.8 g) was used instead of trehalose, an emulsified powder containing oxidized coenzyme Q10 was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 379 nm.

### (Example 3)

Using the same method as in Example 1 except that erythritol (manufactured by Mitsubishi-Kagaku Foods, 2.0 g) was used instead of trehalose, and gum arabic (manufactured by Colloïdes Naturels International; Instant Gum AA, 3.8 g) was added as an aqueous phase component, an emulsified powder containing oxidized coenzyme Q10 was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 397 nm.

### (Example 4)

Using the same method as in Example 1 except that glucose (manufactured by NIHON SHOKUHIN KAKO CO., LTD.; Nisshoku hydrous crystal glucose #70, 5.8 g) was used instead of trehalose, an emulsified powder containing oxidized coenzyme Q10 was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 416 nm.

### (Example 5)

Using the same method as in Example 1 except that fructooligosaccharide (manufactured by Meiji Food Materia Co., Ltd.; Meioligo P, 5.8 g) was used instead of trehalose, an emulsified powder containing oxidized coenzyme Q10 was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 327 nm.

### (Example 6)

Using the same method as in Example 1 except that lactosucrose (manufactured by ENSUIKO Sugar Refining Co., Ltd.; Lactosucrose LS-90P, 5.8 g) was used instead of trehalose, an emulsified powder containing oxidized coenzyme Q10 was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 335 nm.

### (Example 7)

Using the same method as in Example 1 except that maltooligosaccharide (Mitsubishi-Kagaku Foods; oligotose, 5.8 g) was used instead of trehalose, an emulsified powder containing oxidized coenzyme Q10 was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 378 nm.

### (Example 8)

Casein sodium (manufactured by NIPPON SHINYAKU CO., LTD.; Hapuro, 5.3 g), fructooligosaccharide (manufactured by Meiji Food Materia Co., Ltd.; Meioligo P, 5.3 g) and enzymatically decomposed lecithin (manufactured by Cargill; Emultop IP, 1.0 g) was dissolved in distilled water (180 g) at 60°C to give an aqueous solution. Separately, oxidized coenzyme Q10 (manufactured by Kaneka Corporation; Kaneka Coenzyme Q10, 8.4 g) was melted at 60°C and added to the above-mentioned aqueous solution. The mixture was emulsified by POLYTRON (manufactured by KINEMATICA) at 10000 rpm × 10 min to give an oil-in-water emulsified composition. Then, the composition was sprayed and dried using a spray dryer (B-290; Nihon BUCHI K.K.) to give an oxidized coenzyme Q10-containing emulsified powder. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 286 nm.

### (Example 9)

To each of the oxidized coenzyme Q10-containing emulsified powders (10 g) obtained in Examples 1 - 8 were added cellulose (manufactured by NIHON SHOKUHIN KAKO CO., LTD, 9.8 g) and magnesium stearate (Manufactured by Nacalai Tesque, Inc., 0.2 g), and the mixture was stirred for 1 min. According to general production method of tablet, tablets (300 mg per tablet, oxidized coenzyme Q10 content 60 mg) were produced. The appearance of each of the obtained tablets was fine and tableting trouble was absent. In addition, problems such as sticking and the like were not found during the production.

### (Comparative Example 1)

Casein sodium (manufactured by NIPPON SHINYAKU CO., LTD.; Hapuro, 11.6 g) was dissolved in distilled water (180 g) at 60°C to give an aqueous solution. Separately, oxidized coenzyme Q10 (manufactured by Kaneka Corporation; Kaneka Coenzyme Q10, 8.4 g) was melted at 60°C and added to the above-mentioned aqueous solution. The mixture was emulsified by POLYTRON (manufactured by KINEMATICA) at 10000 rpm × 10 min to give an oil-in-water emulsified composition. Then, the composition was sprayed and dried using a spray dryer (B-290; Nihon BUCHI K.K.) to give an oxidized coenzyme Q10-containing emulsified powder. As the property of the obtained powder, the flowability was poor, attachment of the powder to the inside of the apparatus was high, and the recovery rate was less than 60%. The obtained emulsified powder was dissolved in water, and the particle size of the emulsified particles was measured in the same manner as in Example 1. As a result, the median size of the emulsified particles was 398 nm.

### (Comparative Example 2)

Casein sodium (manufactured by NIPPON SHINYAKU CO., LTD.; Hapuro, 5.8 g) and dextrin (manufactured by Matsutani Chemical Industry Co., Ltd.; Pinedex #2, 5.8 g) were dissolved in distilled water (180 g) at 60°C to give an aqueous solution. Separately, oxidized coenzyme Q10 (manufactured by Kaneka Corporation; Kaneka Coenzyme Q10, 8.4 g) was melted at 60°C and added to the above-mentioned aqueous solution at 60°C. The mixture was emulsified by POLYTRON (manufactured by KINEMATICA) at 10000 rpm × 10 min to give an oil-in-water emulsified composition. Then, the composition was sprayed and dried using a spray dryer (B-290; Nihon BUCHI K.K.) to give an oxidized coenzyme Q10-containing emulsified powder. The flowability of the obtained emulsified powder was poor, attachment of the powder to the inside of the apparatus was high, and the recovery rate was less than 60%. The obtained emulsified powder was dissolved in water, and the particle size of the emulsified particles was measured in the same manner as in Example 1. As a result, the median size of the emulsified particles was 562 nm.

### (Example 10)

Using the same method as in Example 1 except that reduced coenzyme Q10 (manufactured by Kaneka Corporation; KANEKA QH, 8.4 g) was used instead of oxidized coenzyme Q10, and operating under a nitrogen atmosphere, an emulsified powder containing reduced coenzyme Q10 was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 382 nm.

### (Example 11)

Using the same method as in Example 8 except that reduced coenzyme Q10 (manufactured by Kaneka Corporation; KANEKA QH, 8.4 g) was used instead of oxidized coenzyme Q10, and operating under a nitrogen atmosphere, an emulsified powder containing reduced coenzyme Q10 was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 276 nm.

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the scope of the present invention as set forth in the appended claims.

## Claims

1. A coenzyme Q10-containing composition comprising 1 - 70 wt % of coenzyme Q10 (A), 15 - 94 wt % of casein (B), and at least 5 wt % of saccharide (C) other than polysaccharides, wherein the saccharide (C) other than polysaccharides is at least one kind selected from the group consisting of erythritol, trehalose, and an oligosaccharide having 3 to 5 sugars.

2. The composition of claim 1, further comprising 0.1 - 20 wt % of surfactant (D).

3. The composition of claim 1 or 2, wherein the weight ratio of coenzyme Q10 (A) and casein (B) is within the range of 1:10 - 5:1 and the weight ratio of casein (B) and saccharide (C) other than polysaccharides is within the range of 1:10 - 10:1.

4. The composition of any one of claims 1 to 3, wherein the casein (B) is casein sodium.

5. The composition of any one of claims 2 to 4, wherein the surfactant (D) is at least one kind selected from the group consisting of glycerol fatty acid esters, sucrose fatty acid esters, sorbitan fatty acid esters, lecithins and saponins.

6. The composition of any one of claims 1 to 5, which is a powder.

7. Non-therapeutic use of the composition of claims 1 to 6 in a food, a cosmetic or a feed.

8. A process of manufacturing a food, a pharmaceutical product, a cosmetic or a feed, comprising adding a composition as defined in any one of claims 1 to 6.

9. A production method of a coenzyme Q10-containing powder comprising 1 - 70 wt % of coenzyme Q10 (A), 15 - 94 wt % of casein (B), and at least 5 wt % of saccharide (C) other than polysaccharides, wherein the saccharide (C) other than polysaccharides is at least one kind selected from the group consisting of erythritol, trehalose, and an oligosaccharide having 3 to 5 sugars, which method comprises preparing an oil-in-water emulsified composition comprising coenzyme Q10 (A) as an oil phase and an aqueous solution containing casein (B) and saccharide (C) other than polysaccharides as an aqueous phase, and removing water.

## Patentansprüche

1. Coenzym Q10 enthaltende Zusammensetzung, umfassend 1 bis 70 Gew.-% Coenzym Q10 (A), 15 bis 94 Gew.-% Casein (B) und wenigstens 5 Gew.-% Saccharid (C), das aber kein Polysaccharid ist, wobei das Saccharid (C), das kein Polysaccharid ist, wenigstens eine Art ist, die aus der Gruppe ausgewählt ist, die aus Erythrit, Trehalose und einem Oligosaccharid mit 3 bis 5 Zuckern besteht.

2. Zusammensetzung gemäß Anspruch 1, weiterhin umfassend 0,1 bis 20 Gew.-% Tensid (D).

3. Zusammensetzung gemäß Anspruch 1 oder 2, wobei das Gewichtsverhältnis von Coenzym Q10 (A) und Casein (B) im Bereich von 1:10 bis 5:1 liegt und das Gewichtsverhältnis von Casein (B) und Saccharid (C), das kein Polysaccharid ist, im Bereich von 1:10 bis 10:1 liegt.

4. Zusammensetzung gemäß einem der Ansprüche 1 bis 3, wobei es sich bei dem Casein (B) um Natriumcaseinat handelt.

5. Zusammensetzung gemäß einem der Ansprüche 2 bis 4, wobei das Tensid (D) wenigstens eine Art ist, die aus der Gruppe ausgewählt ist, die aus Glycerinfettsäureestern, Saccharosefettsäureestern, Sorbitanfettsäureestern, Lecithinen und Saponinen besteht.

6. Zusammensetzung gemäß einem der Ansprüche 1 bis 5, bei der es sich um ein Pulver handelt.

7. Nichttherapeutische Verwendung der Zusammensetzung gemäß den Ansprüchen 1 bis 6 in einem Lebensmittel, einer Kosmetik oder einem Futter.

8. Verfahren zur Herstellung eines Lebensmittels, eines pharmazeutischen Produkts, einer Kosmetik oder eines Futters, umfassend das Hinzufügen einer Zusammensetzung gemäß einem der Ansprüche 1 bis 6.

9. Herstellungsverfahren für ein Coenzym Q10 enthaltendes Pulver, das 1 bis 70 Gew.-% Coenzym Q10 (A), 15 bis 94 Gew.-% Casein (B) und wenigstens 5 Gew.-% Saccharid (C), das aber kein Polysaccharid ist, umfasst, wobei das Saccharid (C), das kein Polysaccharid ist, wenigstens eine Art ist, die aus der Gruppe ausgewählt ist, die aus Erythrit, Trehalose und einem Oligosaccharid mit 3 bis 5 Zuckern besteht, wobei das Verfahren das Herstellen einer Öl-in-Wasser-emulgierten Zusammensetzung, die Coenzym Q10 (A) als Ölphase und eine wässrige Lösung, die Casein (B) und Saccharid (C), das kein Polysaccharid ist, als wässrige Phase umfasst, und das Entfernen des Wassers umfasst.

## Revendications

1. Composition contenant la coenzyme Q10, comprenant 1 à 70 % en poids de coenzyme Q10 (A), 15 à 94 % en poids de caséine (B), et au moins 5 % en poids d'un saccharide (C) autre que des polysaccharides, dans laquelle le saccharide (C) autre que des polysaccharides est d'au moins un type choisi dans le groupe constitué par l'érythritol, le tréhalose, et un oligosaccharide ayant 3 à 5 sucres.

2. Composition selon la revendication 1, comprenant en outre 0,1 à 20 % en poids d'un tensioactif (D) .

3. Composition selon la revendication 1 ou 2, dans laquelle le rapport en poids de la coenzyme Q10 (A) à la caséine (B) est situé dans la plage allant de 1/10 à 5/1 et le rapport en poids de la caséine (B) au saccharide (C) autre que des polysaccharides est situé dans la plage allant de 1/10 à 10/1.

4. Composition selon l'une quelconque des revendications 1 à 3, dans laquelle la caséine (B) est de la caséine sodique.

5. Composition selon l'une quelconque des revendications 2 à 4, dans laquelle le tensioactif (D) est d'au moins un type choisi dans le groupe constitué par les esters d'acide gras et de glycérol, les esters d'acide gras et de saccharose, les esters d'acide gras et de sorbitan, les lécithines, et les saponines.

6. Composition selon l'une quelconque des revendications 1 à 5, qui est une poudre.

7. Utilisation non thérapeutique de la composition selon les revendications 1 à 6 dans un aliment, un cosmétique ou un aliment pour animaux.

8. Procédé de fabrication d'un aliment, d'un produit pharmaceutique, d'un cosmétique ou d'un aliment pour animaux, comprenant l'addition d'une composition telle que définie dans l'une quelconque des revendications 1 à 6.

9. Procédé de production d'une poudre contenant la coenzyme Q10, comprenant 1 à 70 % en poids de coenzyme Q10 (A), 15 à 94 % en poids de caséine (B), et au moins 5 % en poids d'un saccharide (C) autre que des polysaccharides, dans lequel le saccharide (C) autre que des polysaccharides est d'au moins un type choisi dans le groupe constitué par l'érythritol, le tréhalose, et un oligosaccharide ayant 3 à 5 sucres, lequel procédé comprend la préparation d'une composition émulsionnée huile dans l'eau comprenant la coenzyme Q10 (A) en tant que phase huileuse et d'une solution aqueuse contenant la caséine (B) et le saccharide (C) autre que des polysaccharides en tant que phase aqueuse, et l'élimination de l'eau.
